# EUROPEAN PATENT APPLICATION

(11) **EP 2 052 723 A1**
(43) Date of publication of application: **29.04.2009**
(21) Application number: 07792473.6
(22) Date of filing: 13.08.2007
(51) Int. Cl.: A61K 31/496, A61K 9/19, A61K 47/10, A61K 47/12, A61K 47/18, A61K 47/26, A61K 47/34, A61K 47/40, A61P 35/00

(54) **STABLE LYOPHILIZED PREPARATION**

(30) Priority: 14.08.2006 JP 2006221314
(71) Applicant: Eisai R&D Management Co., Ltd., Tokyo 112-8088 (JP)
(72) Inventor: ONAI, Katsumi, Kakamigahara-shi Gifu 501-6195 (JP); YOKOYAMA, Makoto, Kakamigahara-shi Gifu 501-6195 (JP)
(74) Representative: HOFFMANN EITLE
(86) International application number: PCT/JP2007/065830
(87) International publication number: WO 2008/020584

(57) **Abstract**

The present invention provides a lyophilized preparation that has excellent stability over time and excellent solubility when reconstituted, and that contains (8E,12E,14E)-7-((4-cycloheptylpiperazin-1-yl)carbonyl)oxy-3,6,16,21-tetrahydroxy-6,10,12,16,20-pentamethyl-18,19-epoxytricosa-8,12,14-trien-11-olide. The present invention discloses a lyophilized preparation containing 1) (8E,12E,14E)-7-((4-cycloheptylpiperazin-1-yl)carbonyl)oxy-3,6,16,21-tetrahydroxy-6,10,12,16,20-pentamethyl-18,19-epoxytricosa-8,12,14-trien-11-olide or a pharmaceutically acceptable salt thereof, 2) a pH regulator for adjusting a pH of the preparation to between 5 and 8, and 3) at least one of excipients selected from the groups consisting of sugars and sugar alcohols.

## Description

### Technical Field

The present invention relates to a stable lyophilized preparation containing a 12-membered ring macrolide-based compound having an antitumor action.

### Background Art

A 12-membered ring macrolide-based compound represented by formula (1), which is (8E,12E,14E)-7-((4-cycloheptylpiperazin-1-yl)carbonyl)oxy-3,6,16,21-tetrahydroxy-6,10,12,16,20-pentamethyl-18,19-epoxytricosa-8,12,14-trien-11-olide,

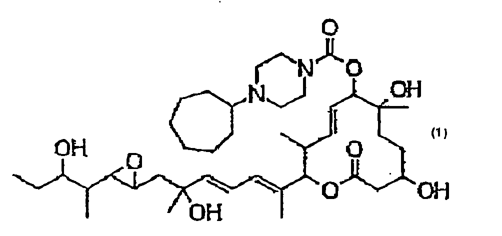

is a compound obtained by the chemical modification of a compound discovered by microbial fermentation (see Patent Document 1). This compound is known to have an action of suppressing VEGF (vascular endothelial growth factor) production in a low-oxygen state *in vitro,* and thereby suppressing angiogenesis produced by cancer, and of suppressing solid tumor cell proliferation *in vivo.*

When the compound represented by the above-mentioned formula (1) is used as an active ingredient, it has to be formulated into various forms for it to be developed as an actual pharmaceutical. The product is formulated as a solid, liquid, etc., according to the properties of the active ingredient.
Patent Document 1: International Publication No. WO 03/099813

### Disclosure of the Invention

### Problems to be Solved by the Invention

The formulation of the compound represented by formula (1) was studied by the inventors, whereupon they found that in the formulation of an injection, the compound represented by formula (1), which is the active ingredient, is not sufficiently stable in solution, and developing an aqueous injection entails a high degree of difficulty. Meanwhile, since the compound represented by formula (1) has good stability in the form of a crystalline powder, its formulation as a packed powder was studied, but because the crystal dissolution of the compound represented by formula (1) is slow, it takes a long time to dissolved into a preparation at the time of its use, among other problems that were found.

In light of the above situation, it is an object of the present invention to provide a lyophilized preparation that has excellent stability over time and excellent solubility when reconstituted, and that contains the compound represented by formula (1) or a pharmaceutically acceptable salt thereof.

### Means for Solving the Problems

As a result of diligent research aimed at solving the above problems, the inventors discovered that a lyophilized preparation that has excellent stability over time and excellent solubility when reconstituted can be obtained by lyophilizing the compound represented by formula (1) or a pharmaceutically acceptable salt thereof, along with a specific pH regulator and a specific excipient. In particular, with a lyophilized preparation, it was discovered that when the excipient is present as an amorphous powder, its stabilizing effect on the lyophilized preparation is especially good. It was also discovered that when citric acid or a salt thereof is used as the pH regulator, the stabilizing effect on the lyophilized preparation will be particularly good. It was also discovered that stability is further enhanced by the addition of glycine, cyclodextrins, or polyethylene glycol as a stabilizer of the preparation. In addition, it was discovered that adding an antioxidant or replacing the head space of the vial or other container with nitrogen helps stabilize the preparation, and this led to the completion of the present invention.

Specifically, it was discovered that the initial object of the present invention could be attained, and the present invention completed, by adopting the following constitution. According to the first aspect of the present invention, there is provided: a lyophilized preparation, comprising:
1) a compound represented by the following formula (1) or a pharmaceutically acceptable salt thereof;
2) a pH regulator for adjusting a pH to between 5 and 8; and
3) at least one of excipients selected from the group consisting of sugars and sugar alcohols.

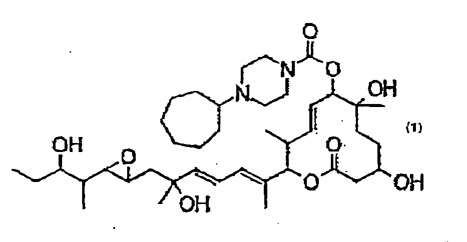

If the pH regulator is phosphoric acid or a salt thereof, however, mannitol is excluded from the excipients.

In the second aspect of the present invention, there is provides : an article comprising: a container; and the lyophilized preparation as above, wherein a head space of the container after being filled with the lyophilized preparation is replaced with nitrogen.

Note that he term "article" as used in present specification refers to a container that includes the lyophilized preparation according to the present invention. Specific examples of the containers may include a vial filled with the lyophilized preparation. Also, in the present invention, the product of dissolving a pH regulator in water or an aqueous solvent (such as a mixture of water and alcohol) is sometimes called a pH regulating solution.

### Advantageous Effects of the Invention

According to the present invention, there is provided a lyophilized preparation that has excellent stability over time and excellent solubility when reconstituted, and that includes (8E,12E,14E)-7-((4-cycloheptylpiperazin-1-yl)carbonyl)oxy-3,6,16,21-tetrahydroxy-6,10,12,16,20-pentamethyl-18,19-epoxytricosa-8,12,14-trien-11-olide.

### Best Mode for Carrying Out the Invention

The following embodiments are illustrative examples of the present invention, and the present invention should not be construed as being limited to these embodiments alone. The present invention can be implemented in various different forms without departing from the gist thereof.

### Compound Represented by formula (1) or Pharmaceutically Acceptable Salt Thereof

The compound represented by formula (1) ((8E,12E,14E)-7-((4-cycloheptylpiperazin-1-yl)carbonyl)oxy-3,6,16,21-tetrahydroxy-6,10,12,16,20-pentamethyl-18,19-epoxytricosa-8,12,14-trien-11-olide) used in the present invention can be synthesized by the method discussed in International Publication No. WO 03/099813, and may be used directly as it is, or in the form of a pharmaceutically acceptable salt thereof.

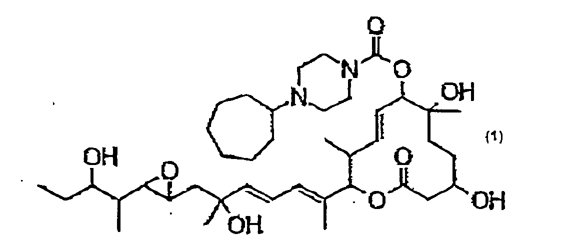

There are no particular restrictions on the term "pharmaceutically acceptable salt" as used in the present invention, it is generally a salt of an acid, examples of which may include hydrochlorides, sulfates, phosphates, citrates, tartrates, methanesulfonates, ethanesulfonates, hydrobromides, and toluenesulfonates.

The lyophilized preparation according to the present invention (hereinafter referred to simply as "this lyophilized preparation") contains as its active ingredient the compound represented by formula (1) or a pharmaceutically acceptable salt thereof, and the content of the compound represented by formula (1) or pharmaceutically acceptable salt thereof in a vial is generally from 0.1 to 100 mg, and preferably from 0.2 to 75 mg, and more preferably from 0.4 to 50 mg, and even more preferably from 0.5 to 30 mg.

pH Regulator for adjusting a pH of the Preparation to between 5 and 8 The pH regulator for adjusting the pH of this lyophilized preparation is one that adjusts the pH of an aqueous solution subjected to lyophilization treatment to between 5 and 8, and preferably between 5.4 and 7.6, and more preferably between 5.8 and 7.2. There are no particular restrictions on the pH regulator for adjusting the pH of the preparation to between 5 and 8 as long as the pH regulator is one that is commonly used in pharmaceutical preparations, examples thereof may include citric acid and salts thereof (such as sodium citrate), tartaric acid and salts thereof (such as sodium tartrate), phosphoric acid and salts thereof (such as sodium dihydrogenphosphate and sodium monohydrogenphosphate), carbonic acid and salts thereof (such as sodium carbonate and sodium hydrogencarbonate), lactic acid and salts thereof (such as sodium lactate), acetic acid and salts thereof (such as sodium acetate), meglumine, sodium hydroxide and the like. It is preferable to use citric acid and the salts thereof (such as sodium citrate), tartaric acid and the salts thereof (such as sodium tartrate), or phosphoric acid and the salts thereof (such as sodium dihydrogenphosphate and sodium monohydrogenphosphate), and more preferably citric acid and the salts thereof (such as sodium citrate), and in any case, an aqueous solution of sodium hydroxide, hydrochloric acid, or the like may be used to adjust the pH of the preparation to between 5 and 8. As listed above, examples of the salts in the pH regulator may include a sodium salt, the pH regulator used in the present invention is not limited to this, and may also include a potassium salt or the like.

There are no particular restrictions on the amount in which the pH regulator is added, it is generally from 0.1 to 300 parts by weight, and preferably from 0.2 to 200 parts by weight, and more preferably from 0.3 to 100 parts by weight, and even more preferably from 0.3 to 50 parts by weight, based on one part by weight of the compound represented by formula (1).

One or More Excipient Selected from the Group Consisting of Sugars and Sugar Alcohols
The excipient used in this lyophilized preparation is one or more types selected from the group consisting of sugars and sugar alcohols. There are no particular restrictions on the sugar, examples thereof may include monosaccharides such as glucose, fructose and the like, and disaccharides such as maltose, lactose, sucrose, trehalose, and the like. Examples of sugar alcohols may include mannitol, erythritol, inositol, sorbitol and the like.

Of these sugars and sugar alcohols, an excipient that is present as an amorphous powder in the lyophilized preparation is desirable because of its good effect of stabilizing the compound represented by formula (1) or pharmaceutically acceptable salt thereof over time. More specifically, examples of monosaccharides may include glucose, fructose and the like, examples of disaccharides may include maltose, lactose, sucrose, trehalose and the like, and examples of sugar alcohols may include inositol, sorbitol and the like. Sucrose is preferable.

With this lyophilized preparation, when the excipient contains two or more types of excipient selected from the groups consisting of sugars and sugar alcohols, the excipient present as an amorphous powder in the lyophilized preparation is contained in an amount of at least 40 % by weight, and preferably at least 50 % by weight, and more preferably at least 60 % by weight, based on a total weight of the excipient.

The amount of the one or more types of excipient selected from the group consisting of sugars and sugar alcohols in this lyophilized preparation is from 2 to 1500 parts by weight, and preferably from 4 to 1000 parts by weight, and more preferably from 6 to 500 parts by weight, and even more preferably from 10 to 300 parts by weight, based on one part by weight of the compound represented by formula (1).

In addition to this lyophilized preparation containing 1) the compound represented by formula (1) or a pharmaceutically acceptable salt thereof, 2) a pH regulator for adjusting the pH of the preparation to between 5 and 8, and 3) at least one excipient selected from the group consisting of sugars and sugar alcohols, this lyophilized preparation may further contain at least one stabilizer selected from the group consisting of cyclodextrins, glycine, and polyethylene glycol.

There are no particular restrictions on the cyclodextrins as the stabilizer used in the present invention, examples thereof may include α-cyclodextrin, β-cyclodextrin, γ-cyclodextrin, partially methylated β-cyclodextrin, dimethyl-β-cyclodextrin, glycosyl-β-cyclodextrin, hydroxypropyl-β-cyclodextrin, and sulfobutyl ether-β-cyclodextrin. It is preferable to use β-cyclodextrin, γ-cyclodextrin, partially methylated β-cyclodextrin, dimethyl-β-cyclodextrin, glycosyl-β-cyclodextrin, hydroxypropyl-β-cyclodextrin, or sulfobutyl ether-β-cyclodextrin. It is more preferable to use β-cyclodextrin, dimethyl-β-cyclodextrin, hydroxypropyl-β-cyclodextrin, or sulfobutyl ether-β-cyclodextrin. It is even more preferable to use hydroxypropyl-β-cyclodextrin.

The polyethylene glycol as the stabilizer used in the present invention is not limited to any particular molecular weight, examples thereof may include polyethylene glycol 200, 300, 400, 500, 600, 700, 800, 900, 1000, 1100, 1200, 1300, 1400, 1450, 1500, 1600, 1700, 1800, 1900, 2000, 2100, 2200, 2300, 2400, 2500, 2600, 2700, 2800, 2900, 3000, 3250, 3350, 3500, 3750, 4000, 4250, 4500, 4750, 5000, 5500, 6000, 6500, 7000, 7500, and 8000. Regardless of its molecular weight, polyethylene glycol has an extremely good effect of stabilizing the compound represented by formula (1), in an example of use as an injection, if we take into account the fact that there are more polyethylene glycols with a low molecular weight, and the fact that, as a characteristic of freeze drying technology, the higher is the molecular weight of an additive, the better is the freeze drying, among other such facts, it is preferable to use polyethylene glycol 700, 800, 900, 1000, 800, 1000, or the like, and polyethylene glycol 600 is especially favorable.

There are no particular restrictions on the amount in which the stabilizer is added, it is generally from 0.2 to 500 parts by weight, and preferably from 0.4 to 400 parts by weight, and more preferably from 0.6 to 300 parts by weight, and even more preferably from 1 to 100 parts by weight, based on one part by weight of the compound represented by formula (1).

This lyophilized preparation further includes an antioxidant. The present invention related to the lyophilized preparation that contains an antioxidant in addition to 1) the compound represented by formula (1) or a pharmaceutically acceptable salt thereof, 2) a pH regulator for adjusting the pH of the preparation to between 5 and 8, and 3) at least one excipient selected from the group consisting of sugars and sugar alcohols. Since there are situations when tiny amounts of oxides of the compound represented by formula (1) are produced, the addition of an antioxidant can suppress the production of these oxides.

There are no particular restrictions on the antioxidant as long as it is one that is generally used in pharmaceutical preparations, examples thereof may include L-cysteine hydrochloride, sodium nitrite, ascorbic acid, citric acid, tocopherol, tocopherol acetate, dibutylhydroxytoluene, sodium hydrogensulfite, alpha-thioglycerol, sodium thioglycolate and the like.

A surfactant, osmotic pressure regulator, preservative, or the like may also be added to this lyophilized preparation. Examples of surfactants may include, but are not limited to, polysorbate, polyoxyethylene polyoxypropylene glycol, polyethylene hydrogenated castor oil, and sorbitan sesquioleate; examples of osmotic pressure regulators may include, but are not limited to, polysorbate, glucose, xylitol, and sorbitol; and examples of preservatives may include, but are not limited to, polysorbate, benzoic acid, ascorbic acid and the like.

Also, this lyophilized preparation is one that contains 1) the compound represented by formula (1) or a pharmaceutically acceptable salt thereof, 2) a pH regulator for adjusting the pH of the preparation to between 5 and 8, and 3) at least one type of excipient selected from the group consisting of sugars and sugar alcohols, to which is added at least one type of stabilizer selected from the group consisting of cyclodextrins, glycine, and polyethylene glycol, and in which a head space of a vial filled with this preparation has been replaced with nitrogen.

The water content of this lyophilized preparation at the time of its manufacture can be controlled by adjusting the drying time and temperature in the freeze drying step. The water content of this lyophilized preparation at the time of its manufacture is generally no more than 2.0%, and preferably no more than 1.7%, and more preferably no more than 1.5%.

This lyophilized preparation provides a good characterization that the compound represented by formula (1) or a pharmaceutically acceptable salt thereof in the preparation has extremely good stability over time and solubility when reconstituted. This lyophilized preparation is lyophilized along with 1) a compound represented by the following formula (1) or a pharmaceutically acceptable salt thereof, 2) a pH regulator for adjusting the pH to between 5 and 8, and 3) at least one type of excipient selected from the group consisting of sugars and sugar alcohols, which gives a lyophilized preparation that has excellent stability over time and excellent solubility when reconstituted. When an excipient is present as an amorphous powder, and particularly when citric acid of a salt thereof is used as a pH regulator, this lyophilized preparation is characterized by a particularly good stabilizing effect. Stability is further enhanced by adding cyclodextrin, glycine, or polyethylene glycol as a stabilizer. Adding an antioxidant, or replacing with nitrogen the head space of a vial filled with this lyophilized preparation, also serves to increase the stability of the lyophilized preparation.

This lyophilized preparation can be manufactured by the following method, for example. 1) the compound represented by formula (1) or a pharmaceutically acceptable salt thereof, 2) a pH regulator for adjusting the pH of the preparation to between 5 and 8, and 3) at least one type of excipient selected from the group consisting of sugars and sugar alcohols, and, if needed, a stabilizer, an antioxidant, etc., are dissolved in water or a suitable aqueous solvent (such as a mixture of water and alcohol), and this solution is subjected to filtration sterilization with a membrane filter or the like. The sterile solution is then aliquoted into vials, trays, or the like and subjected to ordinary freeze drying, which gives a solid powder.

With this lyophilized preparation manufactured as above, decomposition of the compound represented by formula (1) or pharmaceutically acceptable salt thereof over time is suppressed, so the product has excellent stability and solubility when reconstituted.

When the above-mentioned solution is prepared, the compound represented by formula (1) or pharmaceutically acceptable salt thereof, the excipient, and the pH regulator may be dissolved in water or an aqueous solvent (such as a mixture of water and alcohol) according to the known method (the solution obtained by this dissolution will hereinafter be referred to as the "aqueous solution"). The order of dissolution is not particularly related to the type of component, and the components can be dissolved as desired, but because the compound represented by formula (1) or pharmaceutically acceptable salt thereof has excellent solubility at a low pH, it is preferable to use the pH regulator to obtain a low-pH (5 to 7) liquid preparation in which decomposition of the compound represented by formula (1) or pharmaceutically acceptable salt thereof is suppressed, dissolve the compound represented by formula (1) or pharmaceutically acceptable salt thereof, and then adjust to a specific pH. Also, the solution temperature during manufacture may be kept low in order to prevent the compound represented by formula (1) or pharmaceutically acceptable salt thereof from decomposing in the aqueous solution. The concentration of the compound represented by formula (1) or pharmaceutically acceptable salt thereof in the aqueous solution is generally from 0.1 to 10 mg/mL, and preferably from 0.5 to 5 mg/mL, and more preferably from 0.5 to 2 mg/mL.

The concentration of the excipient in the aqueous solution is generally from 10 to 200 mg/mL, and preferably from 20 to 175 mg/mL, and more preferably from 30 to 150 mg/mL.

The pH of the aqueous solution subjected to lyophilization is from 5 to 8, and preferably from 5.4 to 7.6, and more preferably from 5.8 to 7.2. The solution can be adjusted to the desired pH by suitably adding the above-mentioned pH regulator.

The aqueous solution prepared in this manner is preferably pre-frozen below the eutectic point and the glass transition point, after which the inside of the dryer is held at a vacuum while the rack temperature is gradually raised to the primary drying temperature, and primary drying is carried out at this temperature. Upon completion of the primary drying, the rack temperature is raised until the secondary drying temperature is reached, and secondary drying is carried out at this temperature.

The following is a specific method for manufacturing a lyophilized preparation. An aqueous solution containing 1) the compound represented by formula (1) or a pharmaceutically acceptable salt thereof, 2) a pH regulator for adjusting the pH of the preparation to between 5 and 8, and 3) at least one type of excipient selected from the group consisting of sugars and sugar alcohols, and, if needed, a stabilizer, an antioxidant, etc., is sterilized and then aliquoted in a specific amount into vials. Pre-freezing is performed at a temperature of approximately -60°C to -20°C, followed by primary drying under a reduced pressure at approximately - 20°C to 20°C, and then secondary drying is performed under a reduced pressure at approximately 10°C to 40°C, thereby completing the lyophilization. If needed, the head space of the vials is replaced with nitrogen gas, and then the vials are stoppered to obtain a lyophilized preparation. During the pre-freezing, the temperature must be kept low, not rising to the eutectic point or the glass transition point, so that the excipient will not undergo crystallization. It will depend on the mix ratio of the components that make up the excipient, but generally, when a sugar or a sugar alcohol is included, such as when sucrose and/or lactose is included, it is generally preferable for the pre-freezing to be carried out at a temperature of -40°C or lower.

With this lyophilized preparation obtained in this manner, since the excipient is present as an amorphous powder, stability can be maintained over an extended period of time.

Also, this lyophilized preparation has good reconstitution. Specifically, it reconstitutes extremely easily upon the addition of any suitable solvent (reconstituted solution), allowing the pre-freeze drying solution to be reconstructed. Examples of this reconstituted solution may include injection-use distilled water, physiological saline, and common transfusions (such as glucose and amino acid transfusions).

The solution obtained by reconstituting this lyophilized preparation can be administered parenterally as an intravenous injection, a subcutaneous injection, an intramuscular injection, a dropping injection, or another such injection, or as eye drops. This solution obtained by dissolution in injection-use distilled water, physiological saline, transfusion is sufficiently stable. The concentration of the compound represented by formula (1) or pharmaceutically acceptable salt thereof when reconstituted or diluted is generally from 0.001 to 10 mg/mL, and preferably from 0.005 to 5 mg/mL.

### Examples

The present invention will now be described in further detail by giving examples and control examples thereof. These are intended to be illustrative in nature, and the present invention is not limited to or by the following examples, etc. A person skilled in the art will be able to work the present invention by adding various modifications to the examples given below, and such modifications are encompassed by the patent claims.

The additives mentioned in the following examples were in compliance with Japanese Pharmacopoeia IV, Japanese Pharmaceutical Excipients 2005 (JPE), Japanese Pharmaceutical Codex (JPC) 2005, USP/NF XXIII, and other such compendia, or were reagents.

Manufacturing Example 1 (Examination of type of pH regulator, type of one or more excipients selected from the group consisting of sugars and sugar alcohols, and amounts thereof)

### Reference Example 1 and Examples 1 to 6

The lyophilized preparations in Reference Example 1 and Examples 1 to 6 of the present invention were manufactured by the following method, varying the type and amounts of the pH regulator for adjusting the pH of the preparation to between 5 and 8, and of the one or more excipients selected from the group consisting of sugars and sugar alcohols, which were added to the compound represented by formula (1).

A specific amount of citric acid monohydrate or phosphoric acid was dissolved in injection-use distilled water, and a sodium hydroxide solution was used to adjust the pH while a specific amount of excipient (mannitol, sucrose, or lactose) was dissolved, thereby preparing 50 mL of a 50 mM or 100 mM pH regulator (pH 6) containing a sugar or sugar alcohol.

Next, the compound represented by formula (1) was dissolved (1 mg/mL) in 6 mL of these solutions, after which the solutions were filtered (0.22 µm filter), and 1 mL of each resulting solution was aliquoted into vials and lyophilized. The head space in the lyophilized vials was replaced with nitrogen and the vials were stoppered with rubber plugs, after which they were wrapped with aluminum tape, which gave these lyophilized preparations as Reference Example 1 and Examples 1 to 6 (Table 1). Note that the amounts of pH regulator in the preparation filling into one vial were such that 4.9 mg of phosphoric acid (Reference Example 1 and Examples 1 and 2) or 10.5 mg of citric acid monohydrate (Examples 3 to 5) corresponding to 50 mM, while 21.0 mg of citric acid monohydrate (Example 6) corresponded to 100 mM.

Control Examples 1 and 2 were also manufactured. In Control Example 1, 1 mg of the compound represented by formula (1) was measured out and put into a vial while still a powder, the head space of the vial was replaced with nitrogen and the vials were stoppered with a rubber plug, and the vial was wrapped with aluminum tape to produce a powder-filled preparation. In Control Example 2, the lyophilized preparation included the compound represented by formula (1) and a pH regulator for adjusting the pH of the preparation to approximately 6, and was manufactured by the same method as in Examples 3 to 5, except that the specific amount of excipient (mannitol, sucrose, or lactose) was not added.

The properties of the preparations thus manufactured were evaluated. The evaluation categories were the properties when reconstituted immediately after manufacture, and the increase in impurities originating from the compound represented by formula (1) after storage for 2 weeks at 40°C and 75% relative humidity.
The properties when reconstituted immediately after manufacture were evaluated by adding 1 mL of injection-use distilled water and physiological saline to the lyophilized preparations filling into one vial, reconstituting, and evaluating the reconstitution by the Sugimori C method. The Sugimori C method is discussed in Seiyaku Koujou, p. 574, Vol. 6, No. 6, 1986, and is a way to evaluate the reconstitution of an injection that is dissolved at the time of use.

At the same time, the solution pH of the reconstituted preparations was evaluated.
The increase in impurities after storage for 2 weeks at 40°C and 75% relative humidity was evaluated as follows. The amount of impurities in the preparation immediately after manufacture and the amount of impurities in the preparation after storage for 2 weeks at 40°C and 75% relative humidity were each evaluated by high-performance liquid chromatography, and an evaluation was given as the increase (%) as compared to the amount of impurities in the preparation immediately after manufacture. Typical HPLC conditions are shown below.

Column: L-column ODS (4.6 x 150 mm, 5 um, Chemicals Evaluation and Research Institute)
Column temperature: 40°C
Detector: UV/PDA (detection wavelength: 241 nm)
Mobile phase A: acetonitrile/purified water/potassium dihydrogenphosphate/sodium perchlorate (500 mL/4500 mL/ 1.0 g/35 g)
Mobile phase B: acetonitrile/purified water/sodium perchlorate (4500 mL/500 mL/35 g)
Flux: 1 mL/min
Sample solution: 20% acetonitrile
Sample cooler: 5°C
Injection amount: 10 µL (0.2 mg/mL)
Gradient program: B28% (0 min) - B28% (15 min) - B100% (20 min) - B28% (25.01 min) - B28% (33 min)

Note that the increase in impurities was indicated by both the increase (%) in the total amount of impurities and the increase in a main impurity A caused by decomposition of the compound represented by formula (1). The main impurity A is surmised to be one of the two compounds represented by formula (2), or a mixture of both.

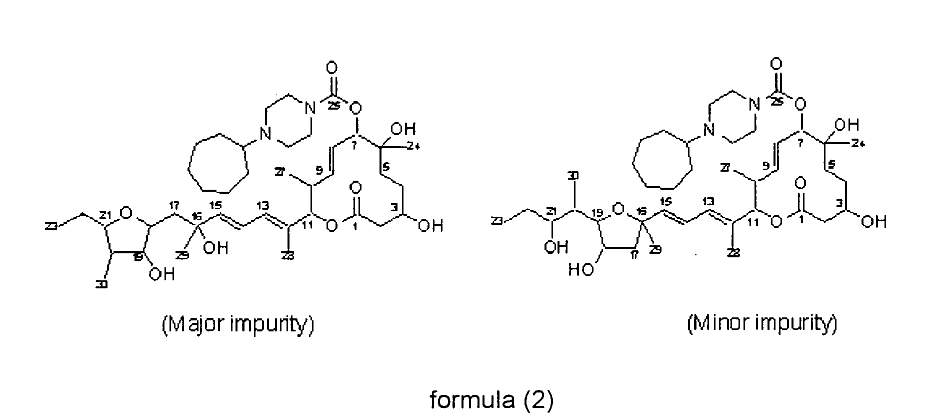

Table 1 shows the lyophilized formulations and properties in Reference Example 1 and Examples 2 to 6.

**TABLE 1**

| | FORMULATION (1 VIAL) | | | PROPERTIES WHEN RECONSTITUTED | | INCREASE IN IMPURITIES AFTER 2 WEEKS OF STORAGE AT 40°C AND 75% RELATIVE HUMIDITY (%) | |
|---|---|---|---|---|---|---|---|
| | COMPOUND REPRESENTED BY FORMULA (1) | pH REGULATOR ¹⁾ | EXCIPIENT | RECONSTITUTION (SECONDS) | pH | INCREASE IN IMPURITY A (%) | INCREASE IN TOTAL AMOUNT OF IMPURITIES (%) |
| REFERENCE EXAMPLE 1 | 1 mg | PHOSPHORIC ACID: 4.9mg | MANNITOL: 50mg | 5 SECONDS OR LESS | 6.11 | 3.10 | 4.90 |
| | | | | | | | |
| EXAMPLE 1 | 1 mg | PHOSPHORIC ACID: 4.9mg | SUCROSE: 100mg | 5 SECONDS OR LESS | 6.21 | 0.99 | 1.06 |
| | | | | | | | |
| EXAMPLE 2 | 1 mg | PHOSPHORIC ACID: 4.9mg | LACTOSE: 100mg | 5 SECONDS OR LESS | 6.11 | 0.82 | 1.30 |
| EXAMPLE 3 | 1 mg | CITRIC ACID MONOHYDRATE: 10.5mg | MANNITOL: 50mg | 10 SECONDS OR LESS | 6.11 | 0.53 | 1.36 |
| | | | | | | | |
| EXAMPLE 4 | 1 mg | CITRIC ACID MONOHYDRATE: 10.5mg | SUCROSE: 100mg | 5 SECONDS OR LESS | 6.21 | 0.45 | 0.61 |
| | | | | | | | |
| EXAMPLE 5 | 1 mg | CITRIC ACID MONOHYDRATE: 10.5mg | LACTOSE: 100mg | 5 SECONDS OR LESS | 6.10 | 0.47 | 1.11 |
| EXAMPLE 6 | 1 mg | CITRIC ACID MONOHYDRATE: 21.0mg | SUCROSE: 100mg | 5 SECONDS 5 SECONDS OR LESS | 6.08 | 0.38 | 0.87 |
| CONTROL EXAMPLE 1 | 1 mg | - | - | DID NOT DISSOLVE | - | 0 | 0.11 |
| | | | | | | | |
| CONTROL EXAMPLE 2 | 1 mg | CITRIC ACID MONOHYDRATE: 10.5mg | - | 5 SECONDS OR LESS | 6.10 | 0.59 | 1.44 |

| | | | | | | | |
|---|---|---|---|---|---|---|---|
| 1): pH WAS ADJUSTED USING A SODIUM HYDROXIDE SOLUTION. | | | | | | | |

### Manufacturing Example 2 (Examination of type of sugar present as amorphous powder)

### Examples 7 and 8

The lyophilized preparations of Examples 7 and 8 of the present invention were manufactured by the following method, varying the type of sugar present as an amorphous powder in the lyophilized preparation, as the one or more types of excipient selected from the group consisting of sugars and sugar alcohols that were added to the compound represented by formula (1).
A specific amount of citric acid monohydrate was dissolved in injection-use distilled water, and a sodium hydroxide solution was used to adjust the pH while a specific amount of excipient (sucrose, trehalose, or maltose) was dissolved, thereby preparing 50 mL of a 50 mM pH regulator (pH 6) containing a sugar. Next, 6 mg of the compound represented by formula (1) was dissolved (1 mg/mL) in 6 mL of these solutions, after which the solutions were filtered (0.22 µm filter), and 1 mL of each resulting solution was aliquoted into vials and lyophilized. The head space in the lyophilized vials was replaced with nitrogen and the vials were stoppered with rubber plugs, after which they were wrapped with aluminum tape, which gave these lyophilized preparations as Examples 4, 7, and 8 (Table 2). The amount of pH regulator in the preparation filling into one vial was 10.5 mg of citric acid monohydrate (Examples 4, 7, and 8), and the amount of excipient (sucrose, trehalose, or maltose) was 100 mg.
The properties of the preparations thus manufactured were evaluated by the same method as described in Manufacturing Example 1. Table 2 shows the lyophilized formulations and properties in Examples 4, 7, and 8.

[Table 2]

**TABLE 2**

| | FORMULATION (1 VIAL) | | | PROPERTIES WHEN RECONSDTITUTED | | INCREASE IN IMPURITIES AFTER 2 WEEKS OF STORAGE AT 40°C AND 75% RELATIVE HUMIDITY (%) | |
|---|---|---|---|---|---|---|---|
| | COMPOUND REPRESENTED BY FORMULA (1) | pH REGULATOR ¹⁾ | EXCIPIENT | RECONSTITUTION (SECONDS) | pH | INCREASE IN IMPURITY A (%) | INCREASE IN TOTAL AMOUNT OF IMPURITIES (%) |
| EXAMPLE 4 | 1 mg | CITRIC ACID MONOHYDRATE: 10.5mg | SUCROSE: 100mg | 5 SECONDS OR LESS | 6.21 | 0.45 | 0.61 |
| EXAMPLE 7 | 1 mg | CITRIC ACID MONOHYDRATE: 10.5mg | TREHALOSE: 100mg | 5 SECONDS OR LESS | 6.07 | 0.55 | 0.92 |
| EXAMPLE 8 | 1 mg | CITRIC ACID MONOHYDRATE: 10.5mg | MALTOSE: 100mg | 5 SECONDS OR LESS | 6.12 | 0.52 | 0.88 |

| | | | | | | | |
|---|---|---|---|---|---|---|---|
| 1): pH WAS ADJUSTED USING A SODIUM HYDROXIDE SOLUTION. | | | | | | | |

### Manufacturing Example 3 (Examination of type of stabilizer, type (molecular weight) of polyethylene glycol, and added amounts)

### Examples 9 to 16

The lyophilized preparations of Examples 9 to 16 of the present invention were manufactured by the following method, by adding a stabilizer to a lyophilized preparation that included 1) the compound represented by formula (1) or a pharmaceutically acceptable salt thereof, 2) a pH regulator for adjusting the pH of the preparation to between 5 and 8 (citric acid monohydrate was used), and 3) at least one type of excipient selected from the group consisting of sugars and sugar alcohols (sucrose was used), and varying the type and added amount of this stabilizer.
A specific amount of citric acid monohydrate was dissolved in injection-use distilled water, and a sodium hydroxide solution was used to adjust the pH while specific amounts of sucrose and a stabilizer were dissolved, thereby preparing 50 mL of a 50 mM pH regulator (pH of 6) containing sucrose and the stabilizer.
Next, 6 mg of the compound represented by formula (1) was dissolved (1 mg/mL) in 6 mL of these solutions, after which the solutions were filtered (0.22 µm filter), and 1 mL of each resulting solution was aliquoted into vials and lyophilized. The head space in the lyophilized vials was replaced with nitrogen and the vials were stoppered with rubber plugs, after which they were wrapped with aluminum tape, which gave these lyophilized preparations as Examples 9 to 11 (Table 3), Examples 12 to 14 (Table 4), and Examples 15 and 16 (Table 5).

Note that the amount of pH regulator in the preparation filling into one vial was 10.5 mg of citric acid monohydrate (Examples 9 to 16), and the amount of sucrose excipient was 100 mg. The stabilizers in Examples 9 to 11 were hydroxypropyl-β-cyclodextrin, glycine, and polyethylene glycol 4000, contained in an amount of 10 mg in one vial of the lyophilized preparation. In Examples 12 to 14, the type (molecular weight) of the polyethylene glycol stabilizer was varied, so that polyethylene glycol 400, polyethylene glycol 600, and polyethylene glycol 4000, respectively, were contained in an amount of 10 mg in one vial of the lyophilized preparation. In Examples 15, 13, and 16, the amount in which polyethylene glycol 600 was added as the stabilizer was varied, so that it was contained in amounts of 5 mg, 10 mg, and 20 mg, respectively, in one vial of the lyophilized preparation.

The properties of the preparaions thus manufactured were evaluated by the same method as described in Manufacturing Example 1. Tables 3, 4, and 5 show the lyophilized formulations and properties in Examples 9 to 11, Examples 12 to 14, and Examples 15 and 16, respectively.

[Table 3]

**TABLE 3**

| | FORMULATION (1 VIAL) | | | | PROPERTIES WHEN RECONSTITUTED | | INCREASE IN IMPURITIES AFTER 2 WEEKS OF STORAGE AT 40°C AND 75% RELATIVE HUMIDITY (%) | |
|---|---|---|---|---|---|---|---|---|
| | COMPOUND REPRESENTED BY FORMULA (1) | pH REGULATOR ¹⁾ | EXCIPIENT | STABILIZER | RECONSTITUTION (SECONDS) | pH | INCREASE IN IMPURITY A (%) | INCREASE IN TOTAL AMOUNT OF IMPURITIES (%) |
| EXAMPLE 4 | 1 mg | CITRIC ACID MONOHYDRATE: 21.0mg | SUCROSE: 100mg | - | 5 SECONDS OR LESS | 6.12 | 0.45 | 0.61 |
| EXAMPLE 9 | 1 mg | CITRIC ACID MONOHYDRATE: 10.5mg | SUCROSE: 100mg | HYDROXYP ROPYL-β-GD: 10mg | 5 SECONDS OR LESS | 6.04 | 0.39 | 0.50 |
| | | | | | | | | |
| EXAMPLE 10 | 1 mg | CITRIC ACID MONOHYDRATE: 10.5mg | SUCROSE: 100mg | GLYCINE: 10mg | 5 SECONDS OR LESS | 6.10 | 0.30 | 0.52 |
| | | | | | | | | |
| EXAMPLE 11 | 1 mg | CITRIC ACID MONOHYDRATE: 10.5mg | SUCROSE: 100mg | PEG4000: 10mg | 5 SECONDS OR LESS | 6.14 | 0.23 | 0.45 |

| | | | | | | | | |
|---|---|---|---|---|---|---|---|---|
| 1): pH WAS ADJUSTED USING A SODIUM HYDROXIDE SOLUTION. | | | | | | | | |

[Table 4]

**TABLE 4**

| | FORMULATION (1 VIAL) | | | | PROPERTIES WHEN RECONSTITUTED | | INCREASE IN IMPURITIES AFTER 2 WEEKS OF STORAGE AT 40°C AND 75% RELATIVE HUMIDITY (%) | |
|---|---|---|---|---|---|---|---|---|
| | COMPOUND REPRESENTED BY FORMULA (1) | pH REGULATOR ¹⁾ | EXCIPIENT | STABILIZER | RECONSTITUTION (SECONDS) | pH | INCREASE IN IMPURITY A (%) | INCREASE IN TOTAL AMOUNT OF IMPURITIES (%) |
| EXAMPLE 4 | 1 mg | CITRIC ACID MONOHYDRATE: 10.5mg | SUCROSE: 100mg | - | 5 SECONDS OR LESS | 6.12 | 0.45 | 0.61 |
| EXAMPLE 12 | 1 mg | CITRIC ACID MONOHYDRATE: 10.5mg | SUCROSE: 00 100mg | PEG4: 10mg | 5 SECONDS OR LESS | 6.05 | 0.19 | 0.56 |
| | | | | | | | | |
| EXAMPLE 13 | 1 mg | CITRIC ACID MONOHYDRATE: 10.5mg | SUCROSE: 00 100mg | PEG6: 10mg | 5 SECONDS OR LESS | 6.07 | 0.11 | 0.35 |
| | | | | | | | | |
| EXAMPLE 14 | 1 mg | CITRIC ACID MONOHYDRATE: 10.5mg | SUCROSE: 000 100mg | PEG4: 10mg | 5 SECONDS OR LESS | 6.10 | 0.26 | 0.38 |

| | | | | | | | | |
|---|---|---|---|---|---|---|---|---|
| 1): pH WAS ADJUSTED USING A SODIUM HYDROXIDE SOLUTION, | | | | | | | | |

[Table 5]

**TABLE 5**

| | FORMULATION (1 VIAL) | | | | PROPERTIES WHEN RECONSTITUTED | | INCREASE IN IMPURITIES AFTER 2 WEEKS OF STORAGE AT 40°C AND 75% RELATIVE HUMIDITY (%) | |
|---|---|---|---|---|---|---|---|---|
| | COMPOUND REPRESENTED BY FORMULA (1) | pH REGULATOR ¹⁾ | EXCIPIENT | STABILIZER | RECONSTITUTION (SECONDS) | pH | INCREASE IN IMPURITY A (%) | INCREASE IN TOTAL AMOUNT OF IMPURITIES (%) |
| EXAMPLE 4 | 1 mg | CITRIC ACID MONOHYDRATE: 10.5mg | SUCROSE: 100mg | - | 5 SECONDS OR LESS | 6.12 | 0.45 | 0.61 |
| EXAMPLE 15 | 1 mg | CITRIC ACID MONOHYDRATE: 10.5mg | SUCROSE: 100mg | PEG600: 5mg | 5 SECONDS OR LESS | 6.04 | 0.35 | 0.62 |
| | | | | | | | | |
| EXAMPLE 13 | 1 mg | CITRIC ACID MONOHYDRATE: 10.5mg | SUCROSE: 100mg | PEG600: 10mg | 5 SECONDS OR LESS | 6.05 | 0.11 | 0.35 |
| | | | | | | | | |
| EXAMPLE 16 | 1 mg | CITRIC ACID MONOHYDRATE: 10.5mg | SUCROSE: 100mg | PEG600: 20mg | 5 SECONDS OR LESS | 6.10 | 0.06 | 0.20 |

| | | | | | | | | |
|---|---|---|---|---|---|---|---|---|
| 1): pH WAS ADJUSTED USING A SODIUM HYDROXIDE SOLUTION. | | | | | | | | |

Manufacturing Example 4 (Examination of pH of preparation depending on addition of pH regulator)

### Examples 17 to 21

The lyophilized preparations of Examples 17 to 21 were manufactured by the following method, varying the pH of the formulation according to the pH regulator that was added.

A specific amount of citric acid monohydrate was dissolved in injection-use distilled water, and a sodium hydroxide solution was used to adjust the pH while specific amounts of sucrose and polyethylene glycol 600 (stabilizer) were dissolved, thereby preparing 50 mL of various 50 mM pH regulators (with a pH of 5.5, 5.7, 5.9, 6.1, or 6.3) containing sucrose and the stabilizer. Next, 6 mg of the compound represented by formula (1) was dissolved (1 mg/mL) in 6 mL of these solutions, after which the solutions were filtered (0.22 µm filter), and 1 mL of each resulting solution was aliquoted into vials and lyophilized. The head space in the lyophilized vials was replaced with nitrogen and the vials were stoppered with rubber plugs, after which they were wrapped with aluminum tape, which gave these lyophilized preparations as Examples 17 to 21 (Table 6). The amount of pH regulator in the preparation filling into one vial was 10.5 mg of citric acid monohydrate (Examples 18 to 21), the amount of sucrose excipient was 100 mg, and the amount of polyethylene glycol 600 stabilizer was 20 mg. The properties of the preparations thus manufactured were evaluated by the same method as described in Manufacturing Example 1.

Table 6 shows the lyophilized formulations and properties in Examples 17 to 21.

**TABLE 6**

| | FORMULATION (1 VIAL) | | | | PROPERTIES WHEN RECONSTITUTED | | INCREASE IN IMPURITIES AFTER 2 WEEKS OF STORAGE AT 40°C AND 75% RELATIVE HUMIDITY (%) | |
|---|---|---|---|---|---|---|---|---|
| | COMPOUND REPRESENTED BY FORMULA (1) | pH REGULATOR ¹⁾ | EXCIPIENT | STABILIZER | RECONSTITUTION (SECONDS) | pH | INCREASE IN IMPURITY A (%) | INCREASE IN TOTAL AMOUNT OF IMPURITIES (%) |
| EXAMPLE 17 | 1 mg | CITRIC ACID MONOHYDRATE: 10.5mg | SUCROSE: PEG600: SUCROSE: 100mg | PEG600: 20mg | 5 SECONDS OR LESS | 5.55 | 0.23 | 0.86 |
| | | | | | | | | |
| EXAMPLE 18 | 1 mg | CITRIC ACID MONOHYDRATE: 10.5mg | SUCROSE: 100mg | PEG600: 20mg | 5 SECONDS OR LESS | 5.80 | 0.13 | 0.48 |
| | | | | | | | | |
| EXAMPLE 19 | 1 mg | CITRIC ACID MONOHYDRATE: 10.5mg | SUCROSE: 100mg | PEG600: 20mg | 5 SECONDS OR LESS | 6.02 | 0.10 | 0.36 |
| | | | | | | | | |
| EXAMPLE 20 | 1 mg | CITRIC ACID MONOHYDRATE: 10.5mg | SUCROSE: 100mg | PEG600: 20mg | 5 SECONDS OR LESS | 6.23 | 0.08 | 0.27 |
| | | | | | | | | |
| EXAMPLE 21 | 1 mg | CITRIC ACID MONOHYDRATE: 10.5mg | SUCROSE: 100mg | PEG600: 20mg | 5 SECONDS OR LESS | 6.44 | 0.06 | 0.21 |

| | | | | | | | | |
|---|---|---|---|---|---|---|---|---|
| 1): pH WAS ADJUSTED USING A SODIUM HYDROXIDE SOLUTION. | | | | | | | | |

The effect that the pH of the preparation resulting from the addition of a pH regulator has on the solubility of the compound represented by formula (1) was also examined.
0.525 g of citric acid monohydrate was dissolved in injection-use distilled water, and a sodium hydroxide solution was used to adjust the pH while 5 g of sucrose and 1 g of polyethylene glycol 600 (stabilizer) were dissolved, thereby preparing 50 mL of various 50 mM pH regulators (with a pH of 5.6, 5.8, 6.0, 6.2, 6.4, or 6.6) containing sucrose and the stabilizer. 3 mL of the pH regulating solution thus prepared was put into a 5 mL vial containing approximately 9 mg of the compound represented by formula (1), and this was stored for 1 hour at 5°C while being stirred. Thereafter, the solution (including a suspension) was filtered (0.22 µm filter), the first 1 mL of the filtrate was discarded, and the solution thus obtained was subjected to high-performance liquid chromatography (HPLC) to measure the concentration of the compound represented by formula (1), which was termed the solubility of the compound represented by formula (1). The pH of the solution was also measured at the same time. The results are given in Table 7.

**[Table 7]**

| pH | Solubility (µg/mL) |
|---|---|
| | Compound represented by formula 1 |
| pH 5.79 | >2435 |
| pH 5.99 | >2311 |
| pH 6.20 | >2265 |
| pH 6.51 | >2717 |
| pH 6.81 | 2571 |
| pH 7.04 | 1798 |

### Manufacturing Example 5 (Examination of nitrogen replacement of the head space of the vial)

### Examples 22 and 23

The lyophilized preparations of Examples 22 and 23 of the present invention were manufactured by the following method, varying whether or not the head space of the vial was replaced with nitrogen.
2.1 g of citric acid monohydrate was dissolved in approximately 140 mL of injection-use distilled water, and a sodium hydroxide solution was used to adjust the pH to 5.6. This solution was cooled to between 2 and 10°C while 20 g of sucrose, 4 g of polyethylene glycol 600, and 200 mg of the compound represented by formula (1) were dissolved. The pH of this solution was adjusted to 6.1 with a sodium hydroxide solution, after which the total volume was brought up to 200 mL with injection-use distilled water. Next, these solutions were filtered (0.22 µm filter), and 1 mL of each resulting solution was aliquoted into vials and lyophilized.
The lyophilized vials were divided into two groups, one in which the head space was replaced with nitrogen, and one in which it was replaced with ordinary air. The vials were stoppered with rubber plugs, after which they were wrapped with aluminum tape, which gave these lyophilized preparations as Examples 22 and 23 (Table 8). The amount of pH regulator in the preparation filling into one vial was 10.5 mg of citric acid monohydrate, the amount of sucrose excipient was 100 mg, and the amount of polyethylene glycol 600 stabilizer was 20 mg.

The properties of the preparations thus manufactured were evaluated. The evaluation categories were the initial water content immediately after manufacture, and the increase in impurities after storage for 2 weeks at 40°C and 75% relative humidity. The initial water content was evaluated using the Karl Fischer water gauge (Mitsubishi Chemical) discussed in Japanese Pharmacopoeia IV.

Table 8 shows the lyophilized formulations and properties in Examples 22 and 23.

[Table 8]

**TABLE 8**

| | FORMULATION (1 VIAL) | | | | | INITIAL WATER CONTENT AT MANUFACTURE (%) | INCREASE IN IMPURITIES AFTER 2 WEEKS OF STORAGE AT 40°C AND 75% RELATIVE HUMIDITY (%) | |
|---|---|---|---|---|---|---|---|---|
| | COMPOUND REPRESENTED BY FORMULA (1) | pH REGULATOR ¹⁾ | EXCIPIENT | STABILIZER | NITROGEN REPLACEMENT | | INCREASE IN IMPURITY A (%) | INCREASE IN TOTAL AMOUNT OF IMPURITIES (%) |
| EXAMPLE 22 | 1 mg | CITRIC ACID MONOHYDRATE: 10.5mg | SUCROSE: 100mg | PEG600: 20mg | YES | 0.41 | 0.07 | 0.24 |
| | | | | | | | | |
| EXAMPLE 23 | 1 mg | CITRIC ACID MONOHYDRATE: 10.5mg | SUCROSE: 100mg | PEG600: 20mg | NO | 0.48 | 0.07 | 0.55 |

| | | | | | | | | |
|---|---|---|---|---|---|---|---|---|
| 1): pH WAS ADJUSTED USING A SODIUM HYDROXIDE SOLUTION. | | | | | | | | |

### Manufacturing Example 6 (Examination of adding an antioxidant)

### Examples 24 to 27

The lyophilized preparations of Examples 24 to 27 of the present invention were manufactured by the following method, varying the added amount of antioxidant.
An antioxidant was added to a composition composed of 1) 1 mg of the compound represented by formula (1), 2) 10.5 mg of a pH regulator for adjusting the pH of the preparation to 6.1 (50 mM citric acid monohydrate was used), 3) 100 mg of at least one type of excipient selected from the group consisting of sugars and sugar alcohols (sucrose was used), and 4) 20 mg of a stabilizer (polyethylene glycol 600), and the effect on the stability of the compound represented by formula (1) was examined. L-Cysteine hydrochloride monohydrate was used as the antioxidant, and it was added to the preparation in four levels: 0 mg (not added), 0.01 mg, 0.05 mg, or 0.10 mg.
Specifically, 0.525 g of citric acid monohydrate was dissolved in injection-use distilled water, and a sodium hydroxide solution was used to adjust the pH while 5 g of sucrose, 1 g of polyethylene glycol 600, and various amounts (0 g, 0.005 g, 0.0025 g, and 0.0005 g) of L-cysteine hydrochloride monohydrate were dissolved, thereby preparing 50 mL of various 50 mM pH regulators (with a pH of 6.1) containing sucrose, polyethylene glycol 600, and L-cysteine hydrochloride monohydrate. Next, 6 mg of the compound represented by formula (1) was dissolved (1 mg/mL) in 6 mL of these solutions, after which the solutions were filtered (0.22 µm filter), and 1 mL of each resulting solution was aliquoted into vials and lyophilized. The head space in the lyophilized vials was replaced with nitrogen and the vials were stoppered with rubber plugs, after which they were wrapped with aluminum tape, which gave these lyophilized preparations as Examples 25 to 28 (Table 9).

The properties of the preparations thus manufactured were evaluated. The evaluation categories were the increase in impurities after storage for 2 weeks at 40°C and 75% relative humidity. The increase in impurities was indicated by both the total amount of increased impurities and the amount of increased oxides caused by oxidative decomposition of the compound represented by formula (1), as a ratio of the increase (%) in the total amount of initial impurities immediately after manufacture and the amount of oxides caused by oxidative decomposition of the compound represented by formula (1).

Table 9 shows the lyophilized formulations and properties in Examples 24 to 27.

[Table 9]

**TABLE 9**

| | FORMULATION (1 VIAL) | | | | | AMOUNT OF INITIAL OXIDES AT MANUFACTURE (%) | INCREASE IN IMPURITIES AFTER 2 WEEKS OF STORAGE AT 40°C AND 75% RELATIVE HUMIDITY (%) | |
|---|---|---|---|---|---|---|---|---|
| | COMPOUND REPRESENTED BY FORMULA (1) | pH REGULATOR | EXCIPIENT ¹⁾ | STABILIZER | ANTIOXIDANT | | INCREASE IN AMOUNT OF OXIDES (%) | INCREASE IN TOTAL AMOUNT OF IMPURITIES (%) |
| EXAMPLE 24 | 1 mg | CITRIC ACID MONOHYDRATE: 10.5mg | SUCROSE: 100mg | PEG600: 20mg | - | 0.12 | 0.13 | 0.32 |
| | | | | | | | | |
| EXAMPLE 25 | 1 mg | CITRIC ACID MONOHYDRATE: 10.5mg | SUCROSE: 100mg | PEG600: 20mg | L-CYSTEINE HYDROCHLORIDE MONOHYDRATE: 0.01gm | 0.06 | 0.01 | 0.18 |
| | | | | | | | | |
| EXAMPLE 25 | 1 mg | CITRIC ACID MONOHYDRATE: 10.5mg | SUCROSE: 100mg | PEG600: 20mg | L-CYSTEINE HYDROCHLORIDE MONOHYDRATE: 0.05gm | 0.06 | -0.01 | 0.18 |
| | | | | | | | | |
| EXAMPLE 25 | 1 mg | CITRIC ACID MONOHYDRATE: 10.5mg | SUCROSE: 100mg | PEG600: 20mg | L-CYSTEINE HYDROCHLORIDE MONOHYDRATE: 0.10gm | 0.05 | -0.01 | 0.18 |

| | | | | | | | | |
|---|---|---|---|---|---|---|---|---|
| 1): pH WAS ADJUSTED USING A SODIUM HYDROXIDE SOLUTION. | | | | | | | | |

Manufacturing Example 7 (Examination of the water content of lyophilized preparation)
The water content of the lyophilized preparation was examined to see what effect it has on the stability of the compound represented by formula (1). First, the vials (pH of 6.23) manufactured with nitrogen replacement in Example 20 were opened and allowed to absorb moisture while the change in weight was monitored under controlled humidity, after which the water content of each was measured with a Karl Fischer water gauge, and these results were termed the initial water content at manufacture (%) (five levels: 0.48%, 0.66%, 1.20%, 1.64%, and 2.19%). These were again stoppered with rubber plugs and wrapped with aluminum tape, which gave samples having various initial water contents. These samples were stored for 2 weeks at 40°C and 75% relative humidity, and the increase in impurities was evaluated.
Table 10 shows the lyophilized formulations and properties.

[Table 10]

**TABLE 10**

| FORMULATION (1 VIAL) | | | | | INITIAL WATER CONTENT AT MANUFACTURE (%) | INCREASE IN IMPURITIES AFTER 2 WEEKS OF STORAGE AT 40°C AND 75% RELATIVE HUMIDITY (%) | |
|---|---|---|---|---|---|---|---|
| COMPOUND REPRESENTED BY FORMULA (1) | pH REGULATOR ¹⁾ | EXCIPIENT | STABILIZER | NITROGEN REPLACEMENT | | INCREASE IN IMPURITY A (%) | INCREASE IN TOTAL AMOUNT OF IMPURITIES (%) |
| 1 mg | CITRIC ACID MONOHYDRATE: 10.5mg | SUCROSE: 100mg | PEG600: 20mg | NO | 0.48 | 0.07 | 0.55 |
| | | | | | | | |
| 1 mg | CITRIC ACID MONOHYDRATE: 10.5mg | SUCROSE: 100mg | PEG600: 20mg | NO | 0.66 | 0.06 | 0.53 |
| | | | | | | | |
| 1 mg | CITRIC ACID MONOHYDRATE: 10.5mg | SUCROSE: 100mg | PEG600: 20mg | NO | 1.20 | 0.05 | 0.47 |
| | | | | | | | |
| 1 mg | CITRIC ACID MONOHYDRATE: 10.5mg | SUCROSE: PEG600: SUCROSE: 100mg | PEG600: 20mg | NO | 1.64 | 0.05 | 0.64 |
| | | | | | | | |
| 1 mg | CITRIC ACID MONOHYDRATE: 10.5mg | SUCROSE: PEG600: SUCROSE: 100mg | PEG600: 20mg | NO | 2.19 | 0.06 | 0.87 |

| | | | | | | | |
|---|---|---|---|---|---|---|---|
| 1): pH WAS ADJUSTED USING A SODIUM HYDROXIDE SOLUTION. | | | | | | | |

A tendency was confirmed for decomposition to accelerate if the initial water content at manufacture exceeds 1.5%.

### Advantageous Effects

According to the present invention, it is possible to manufacture a lyophilized preparation that includes the compound represented by formula (1) and that has excellent stability over time and excellent solubility when reconstituted.

### 1. Effect on the reconstitution and stability of the lyophilized preparation containing a pH regulator, an excipient (such as sugars and sugar alcohols), and the compound represented by formula (1) (Manufacturing Examples 1 and 2)

With the preparations serving as a control examples in Manufacturing Example 1 (shown in Table 1), the crystalline compound represented by formula (1) was stable when it was a powder-filled preparation (Control Example 1), but reconstitution was extremely poor, and the preparation did not dissolve. With the lyophilized preparation of Control Example 2, which contained the compound represented by formula 1 and a pH regulator for adjusting the pH of the preparation to 6, the reconstitution was good enough for use in an injection, but an increase was confirmed in the total amount of impurities and impurity A (the main decomposition product).

Meanwhile, when the preparations according to Examples 1 to 6 in Manufacturing Example 1 of the present invention are the lyophilized preparations containing 1) the compound represented by formula (1), 2) a pH regulator for adjusting the pH of the preparation to 6, and 3) at least one type of excipient selected from the group consisting of sugars and sugar alcohols, and an excipient other than mannitol is added, this suppresses an increase in the total amount of impurities and impurity A (the main decomposition product) as compared to Control Example 2, an increase in stability was noted, and reconstitution was also good.

This stabilizing effect was particularly pronounced when a sugar (excipient) was present as an amorphous powder, except with preparations containing mannitol (Reference Example 1, Example 3), and was best off all when sucrose is used as an excipient (Example 4). When citric acid monohydrate (10.5 mg in Example 5, 21.0 mg in Example 6) was used as a pH regulator, the decomposition suppression effect was particularly strong, and in particular the production of impurity A (the main decomposition product) was markedly suppressed.

Also, the preparations according to Examples 7 and 8 in Manufacturing Example 2 of the present invention (shown in Table 2) were the lyophilized preparations similarly containing sucrose, to which were variously added trehalose or maltose, which are sugars present as an amorphous powder in the lyophilized preparation, and in both cases the increase in the total amount of impurities and impurity A (the main decomposition product) was suppressed just as in Example 4, and stability was improved.

It is clear that a pH regulator that adjusts the pH of the preparation to between 5 and 8 (such as citric acid monohydrate) and an excipient (such as sugars and sugar alcohols), and especially a sugar present as an amorphous powder, improved the reconstitution and stability of the lyophilized preparation containing the compound represented by formula (1).

### 2. Effect on the reconstitution and stability of the lyophilized preparation to which the stabilizer has been further added (Manufacturing Example 3)

The preparations according to Examples 9 to 16 in Manufacturing Example 3 of the present invention were the lyophilized preparations containing 1) the compound represented by formula (1), 2) a pH regulator for adjusting the pH of the preparation to between 5 and 8 (citric acid monohydrate was used), and 3) at least one type of excipient selected from the group consisting of sugars and sugar alcohols (sucrose was used), in which the type of stabilizer added and the added amount were varied.
As shown in Table 3, the stabilizing effect was checked by adding as a stabilizer hydroxypropyl-β-cyclodextrin (Example 9), glycine (Example 10), polyethylene glycol (Example 11), etc. In particular, when polyethylene glycol 4000 (molecular weight of 3350) was added (Example 11), a pronounced suppression of the production of impurity A (the main decomposition product) was confirmed, and reconstitution was also good. Also examined was the relationship between the stabilizing effect and the type of polyethylene glycol (molecular weight of 400, 600, or 4000), which has the best stabilizing effect (Examples 12 to 14). As a result, the decomposition of the compound represented by formula (1) was suppressed by the addition of polyethylene glycols of all three molecular weights (Table 4). The stabilizing effect was particularly good with polyethylene glycol 600.
The relationship between the stabilizing effect of polyethylene glycol 600 and the amount in which it was added was also examined. As shown in Table 5, it was found that polyethylene glycol 600 (whose molecular weight is 600) suppressed the decomposition of the compound represented by formula (1), and in particular the production of impurity A (the main decomposition product) was seen to be suppressed.
It was confirmed that the addition of the stabilizer to this lyophilized preparation, and particularly the addition of polyethylene glycol or the like, increased the stability of the lyophilized preparation in proportion to the amount added, and also resulted in good reconstitution.

### 3. Effect on the stability (Manufacturing Example 4) and solubility of the compound represented by formula (1) of the pH of the preparation according to the addition of a pH regulator

The preparations according to Examples 17 to 21 in Manufacturing Example 4 of the present invention were preparations in which a citric acid monohydrate pH regulator was used to adjust the pH of the lyophilized preparation containing the compound represented by formula (1) to 5.5, 5.7, 5.9, 6.1, or 6.3, as shown in Table 6. It was found that at a pH of 5.5 or higher, the higher the pH, the more decomposition of the compound represented by formula 1 was suppressed and the more stable was the preparation.
The effect of the pH of the preparation produced by the addition of a pH regulator on the solubility of the compound represented by formula (1) was examined. As a result, as shown in Table 8, solubility was high when the pH was in a low range of from 5.79 to 7.04, but solubility decreased dramatically when the pH was in a high range. The pKa of the compound represented by formula (1) is approximately 7.9, the decrease in solubility is pronounced until a pH of around 8, and the pH range that can be used for injection purposes is also limited in terms of solubility, so sufficient solubility must be ensured.
Since the pH (5 to 8) of the preparation produced by the pH regulator in this lyophilized preparation affects the stability and solubility of the compound represented by formula (1), it was found that the stability and reconstitution of the lyophilized preparation can be improved by adjusting the pH of the preparation by striking a good balance between stability and solubility.

### 4. Effect of replacing the head space of a vial with nitrogen on the stability of the compound represented by formula (1) (Manufacturing Example 5)

The effect that replacement of the head space of a vial has on the stability of the compound represented by formula (1) in the lyophilized preparation was examined. As a result, there was far less decomposition in Example 22 in which nitrogen replacement was performed, than in Example 23 in which it was not, even if the initial water content at manufacture in Example 22 is substantially the same as that in Example 23 as shown in Table 8.
It was found that the stability of the lyophilized preparation can be improved, and good reconstitution can be ensured, by replacing the head space of the vial filled with this lyophilized preparation with nitrogen.

### 5. Effect of the addition of an antioxidant on the stability of the compound represented by formula (1) (Manufacturing Example 6)

As shown in Table 9, it was confirmed that oxides were produced immediately after manufacture in the lyophilized preparation of the compound represented by formula (1) containing a stabilizer, and that these oxides increased during stability testing. In particular, with a preparation containing polyethylene glycol, an increase in the amount of initial oxides at manufacture and an attendant increase in the total amount of impurities were confirmed (Example 24). Meanwhile, with the preparation in which L-cysteine hydrochloride monohydrate was added as an antioxidant, the addition of just a tiny amount suppressed the increase in the amount of oxides and the attendant increase in the total amount of impurities (Examples 25 to 27).
It was found that with this lyophilized preparation, and particularly when the stabilizer is added, the stability of the lyophilized preparation can be increased by adding a tiny amount of the antioxidant.

### 6. Effect of the water content of the lyophilized preparation on the stability of the compound represented by formula (1).

It can be seen from the results in Table 10 that with this lyophilized preparation, the higher is the water content in the lyophilized preparation, the more the stability of the compound represented by formula (1) decreases, and in particular, the water content of the preparation must be kept to 2.0 wt% or less, and preferably 1.5 wt% or less, which increases the stability of the lyophilized preparation.

### Industrial Applicability

The present invention provides the lyophilized preparation excellent stability over time and excellent solubility when reconstituted, and that includes (8E, 12E, 14E)-7-((4-cycloheptylpiperazin-1-yl)carbonyl)oxy-3,6,16,21-tetrahydroxy-6,10,12,16,20-pentamethyl-18,19-epoxytricosa-8,12,14-trien-11-olide.

## Claims

1. A lyophilized preparation, comprising:
1) a compound represented by the following formula (1) or a pharmaceutically acceptable salt thereof;
2) a pH regulator for adjusting a pH to between 5 and 8; and
3) at least one of excipients selected from the group consisting of sugars and sugar alcohols.

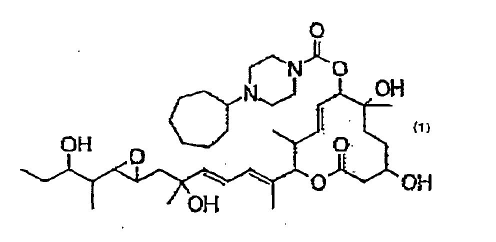

2. The lyophilized preparation according to Claim 1, wherein the excipient is present as an amorphous powder in the lyophilized preparation.

3. The lyophilized preparation according to Claim 1 or 2, wherein the excipient is sucrose.

4. The lyophilized preparation according to any one of Claims 1 to 3, wherein the pH regulator is citric acid or a salt thereof.

5. The lyophilized preparation according to any one of Claims 1 to 4, wherein the pH regulator is contained in an amount of from 0.1 to 300 parts by weight based on one part by weight of the compound represented by the formula (1).

6. The lyophilized preparation according to any one of Claims 1 to 5, further comprising at least one of stabilizers selected from the group consisting of cyclodextrins, glycine, and polyethylene glycol.

7. The lyophilized preparation according to Claim 6, wherein the stabilizer is contained in an amount of from 0.2 to 500 parts by weight based on one part by weight of the compound represented by formula (1).

8. The lyophilized preparation according to Claim 6 or 7, further comprising an antioxidant.

9. The lyophilized preparation according to any one of Claims 1 to 8, wherein a water content of the lyophilized preparation is no more than 2.0 % by weight.

10. The lyophilized preparation according to any one of Claims 1 to 9, wherein the lyophilized preparation is an injection.

11. An article comprising:
a container; and
the lyophilized preparation according to any one of Claims 1 to 10, wherein a head space of the container after being filled with the lyophilized preparation is replaced with nitrogen.
